# EUROPEAN PATENT SPECIFICATION

(11) **EP 0 848 937 B1**
(45) Date of publication and mention of the grant of the patent: **09.10.2002**
(21) Application number: 97122411.8
(22) Date of filing: 18.12.1997
(51) Int. Cl.: A61F 13/02

(54) **Apparatus for manufacturing medicinal patches for percutaneous administration**
Vorrichtung zur Herstellung von medizinischen Pflastern zur perkutanen Verabreichung
Appareil pour la fabrication de pansements médicals pour administration percutanée

(30) Priority: 21.12.1996 KR 9669415
(43) Date of publication of application: 24.06.1998
(73) Proprietor: Sunkyong Industries Co., Suwon-si, Kyungki-do 440-745 (KR); Sunkyong Pharmaceuticals Ltd., Ansan-si, Kyungki-do 425-100 (KR)
(72) Inventor: Uhm, Ki Ahn, 1301 1dong Youngnamvilla, Suwon-si, Kyungki-do, Seoul 440-290 (KR); Min, Dong Sun, Seocho-ku, Seoul, 137-130 (KR)
(74) Representative: Koepe, Gerd L., Dipl.-Chem.

(56) References cited:
- EP-A- 0 571 700
- WO-A-92/19451
- WO-A-94/21207
- US-A- 2 680 943

## Description

### 1. Field of the Invention

The present invention relates to an apparatus for manufacturing medicinal patch for percutaneous administration and more particularly it relates to a main processing unit which performs processing steps such as half die cutting, mutual bonding and die cutting on the base film strip and adhesive tape strip both of which are main stuffs to be used in making the patch. Medicinal patches for percutaneous administration need capability to fulfill their physical requirements, that is, they have such capability as to adhere to the skin of the knee or elbow securely and elastically for a long period of time even during bath or shower bath, and be not easily contaminated by the dirt without loss of medicinal properties.

The patch provided by using the inventive apparatus can be accomplished by attaching a base film which contains medicinal properties over an adhesive tape through a series of manufacturing steps of the present invention. The base film which is used in making the patch of the invention has a structure of lamination of a separate liner, a medicament layer, a first elastic film layer and a first release liner in order; and the adhesive tape which is also used in making the patch of the invention has a structure of lamination of a second elastic film, an adhesive layer and a second release liner in order.

### 2. Discussion of Related Art

It has been reported that piroxicam, a non-steroidal anti-inflammatory and analgesic agent, shows competent efficacy against acute pain due to rheumatoid arthritis, osteoarthritis, and gout etc. when applied by oral administration but causes unfavorable side effects such as gastroenteric disorder, gastric or duodenal ulcer, and the like. In this sense, it is considerably advisable to apply piroxicam by other than oral administration, that is, such as by percutaneous administration.

In the medicinal patch for percutaneous administration, the preparations of its medicinal substrate are of course a matter of primary importance, and the physical characteristics of the patch are by no means the least importance if we are to achieve the desired efficacy of drug over the percutaneous administration. That is, aparting from what the composition of the medicinal substrate contained in the patch, the patch must securely adhere to an articulation such as the knee or elbow maintaining its elasticity even during bath or shower bath, and should be free from contamination and loss of medicinal properties. Further, the medicinal patch preferably has a round or oval shape and is thin and small so as to facilitate topical application thereof. However, conventional medicinal patches in which medicaments are simply applied over a non-woven fabric or textile fail to meet the above requirements.

WO-A-92/19451 discloses a method and apparatus for forming a transdermal drug device. Cutting is progressively achieved along a line coplanar with the axes of a cutting roller and anvil roller counter-rotating in proximity to each other.

### SUMMARY OF THE INVENTION

Accordingly, the present invention is directed to provide an apparatus for manufacturing medicinal patch for percutaneous administration that is thin and small in an oval or round shape to be securely and elastically adhered to the skin of the human body for a long period of time.

To achieve these and other advantages and in accordance with the purpose of the present invention, as embodied and broadly described, there is disclosed an apparatus for manufacturing a medicinal patch for percutaneous administration comprising the following elements:
a half-die cutting roller(a second half-die cutting roller) to ovally or roundly half die cut the medicament layer and first elastic film layer simultaneously laminated on a separate liner of a base film, said half-die cutting roller having large-diameter cylindrical parts, a small-diameter cylindrical part formed between said large-diameter cylindrical parts and a plurality of ovally or roundly shaped kiss cutting blades equidistantly installed on the outer surface of said small-diameter cylindrical part.
a scrap winding roller around which a scrap of the medicament and first elastic film layers produced by half-die cutting is wound for removal;
a pressing means to press the adhesive tape laminated the base film on which ovally or roundly shaped medicament and first elastic film layers are formed by the second half-die cut so as to attach these with each other;
another half-die cutting roller(a third half-die cutting roller) which has a plurality of ovally or roundly half-die cutting blades equidistantly installed on its outer surface of small-diameter cylindrical part, performing continuous half-die cutting on regions of the adhesive tape attached to the base film into an oval or round shape, corresponding to the second half-die cut regions of the medicament and first elastic film layers;
a roller on which a scrap of the adhesive tape produced by the third half-die cutting is wound for removal;
a die cutting roller which has a plurality of quadri-shaped die cutting blades equidistantly installed on its outer surface, performing a continuous die cutting of a resultant strip which has the separate liner, the oval or circular shaped medicament and first elastic film layers and the oval or circular shaped adhesive and second elastic film layers, into a quadri-shaped shape;
a roller on which a scrap of the resultant strip created by the die cutting is wound for removal;
a main die roller having a plurality of vacuum holes on its outer surface for drawing up the separate liner of the base film, and with the outer surface thereof large diameter parts of all the half die cut rollers and die cut roller being in rollably contact; and,
a vacuum pad roller having a plurality of rubber sucking means on its outer surface for drawing up the finished products from the main die roller and transferring them to a conveyor.

It is to be understood that both the foregoing general description and the following detailed description are exemplary and explanatory and are intended to provide further explanation of the invention as claimed.

### BRIEF DESCRIPTION OF THE ATTACHED DRAWINGS

Figs. 1(a) and 1(b) are each a plan view of a medicinal patch provided in accordance with the present invention, and an enlarged-sectional view thereof as taken along the line a - a of Fig. 1(a).

Fig. 2(a) shows a perspective view of a base film roll used for making the inventive medicinal patch for percutaneous administration, and a sectional view thereof as taken along the line b - b.

Fig. 2(b) shows a perspective view of an adhesive tape roll used for the inventive medicinal patch for percutaneous administration, and a sectional view thereof as taken along the line c - c.

Fig. 3 schematically depicts steps of manufacturing the medicinal patch for percutaneous administration in accordance with the present invention.

Figs. 4(a) to 4(m) are each plan and sectional views of respective base film strip and/or adhesive tape strip or its scrap to be removed according to the steps of manufacture the medicinal patch in accordance with the present invention.

Fig. 5 is a front view of an apparatus for manufacturing medicinal patch for percutaneous administration in accordance with the present invention.

Fig. 6 shows an enlarged main part of the apparatus for manufacturing medicinal patch for percutaneous administration in accordance with the present invention.

Fig. 7 is a perspective view of the part B shown in Fig. 6.

Fig. 8 shows sectional views of main die roller and vacuum pad roller.

### DETAILED DESCRIPTION OF PREFERRED EMBODIMENT

Reference will now be made in detail to the preferred embodiments of the present invention, examples of which are illustrated in the accompanying drawings. Like reference numerals denote like reference parts throughout the specification and the drawings.

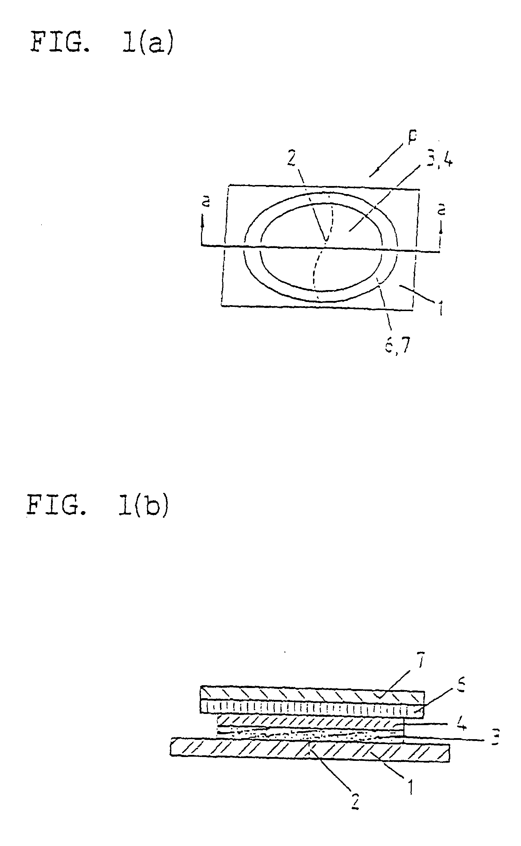

A preferable medicinal patch which obviates the drawbacks of the conventional art and fulfills the physical requirement of a transdermal skin patch is depicted in Figs. 1(a) and 1(b).

Fig. 1(a) is a plan view of the medicinal patch provided according to the present invention, and Fig. 1(b) is an enlarged-sectional view thereof as taken along the line a - a of Fig. 1(a). Reference numeral 1 designates a separate liner that is to be finally removed when the patch P is applied to the skin of the human body. The separate liner 1, being used as a releaser at the time of use, is favorably formed by coating silicon on such the resin as polyethylene terephthalate (PET) or paper. As can be seen from Fig. 1(b) which shows a section of the patch, on the separate liner 1 are deposited a medicament layer 3, a first elastic film layer 4, an adhesive layer 6 and a second elastic film layer 7 in order. The first elastic film layer 4 has an elasticity of more than about 100% and thickness of 5 to 300 micrometers and is preferably made from polyurethane (PU), polyethylene terephthalate (PET) or styrene iso-styrene (SIS), and the like.

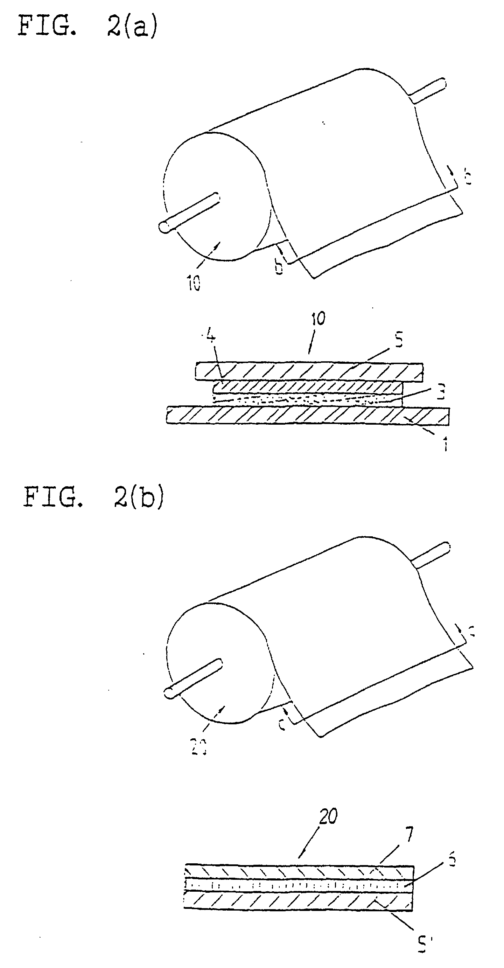

A base film 10 which is used as one of main stuffs in making the patch P of the invention is shown in Fig. 2(a) and has a structure of lamination of a separate liner 1, a medicament layer 3, a first elastic film layer 4 and a first release liner 5 in order. A adhesive tape 20 which is also used as another main stuff in making the patch of the invention has a structure of lamination of a second release liner 5', an adhesive layer 6 and a second elastic film 7 in order as can be seen in Fig. 2(b). The base film 10 and the adhesive tape 20 undergo a series of processing steps such as a few times half-die cutting, mutual bonding, die cutting and drawing-up etc. with the help of the apparatus of the present invention.

A method of manufacturing the base film 10 has been fully described in another korean patent application filed by us on the same date of priority of the present invention, entitled "Method of manufacturing a base film for a transdermal skin patch and an apparatus for making the same". Since the method of manufacturing the base film 10 itself is another invention and not material to understand the present invention, the detailed description thereabout is omitted in this specification. Furthermore, the description about a method of manufacturing the adhesive tape 20 itself is also omitted for the same reason.

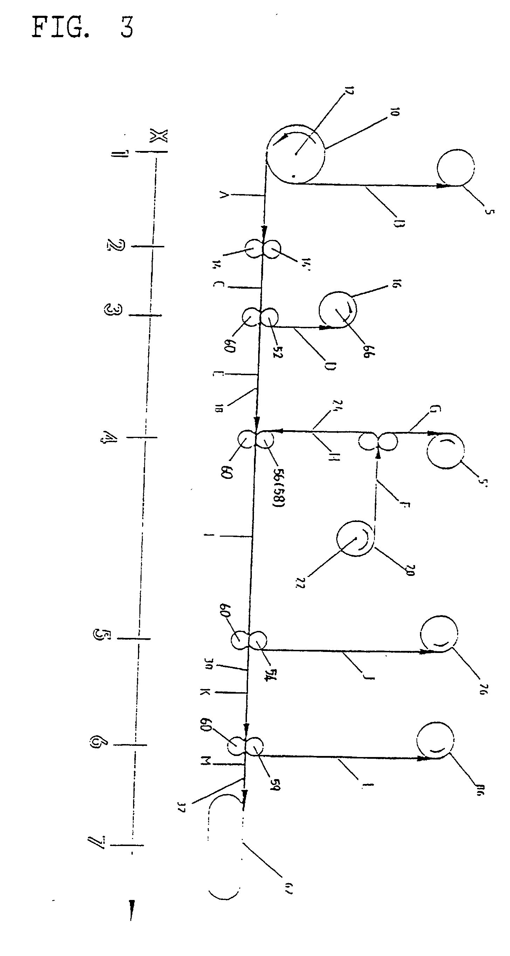

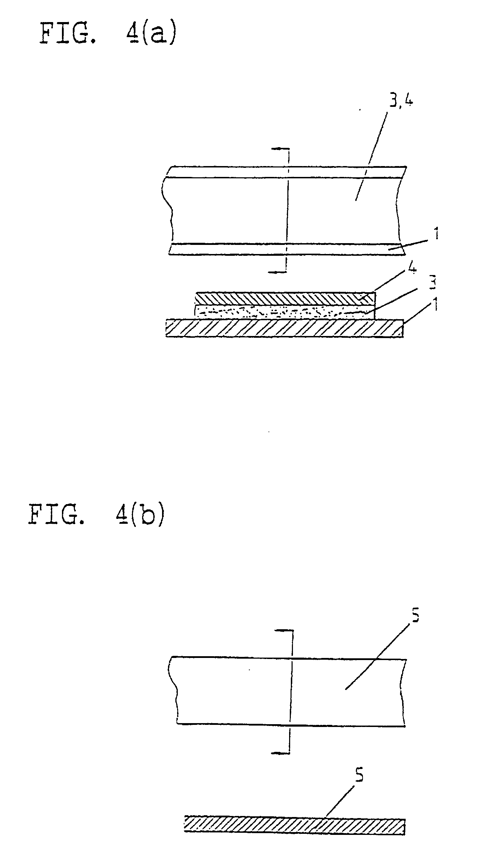

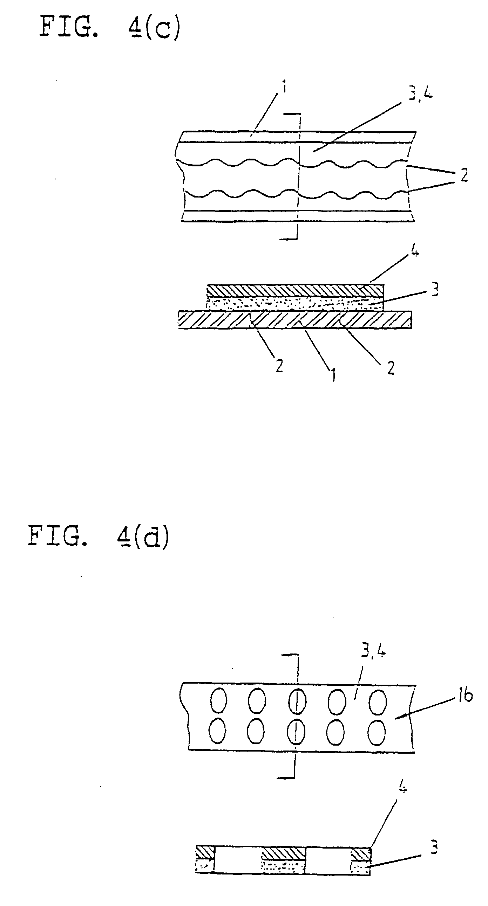

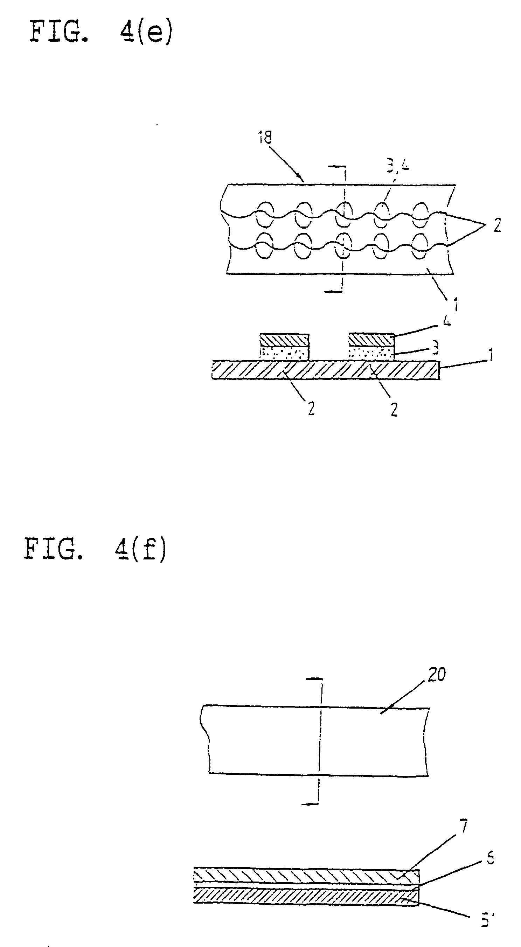

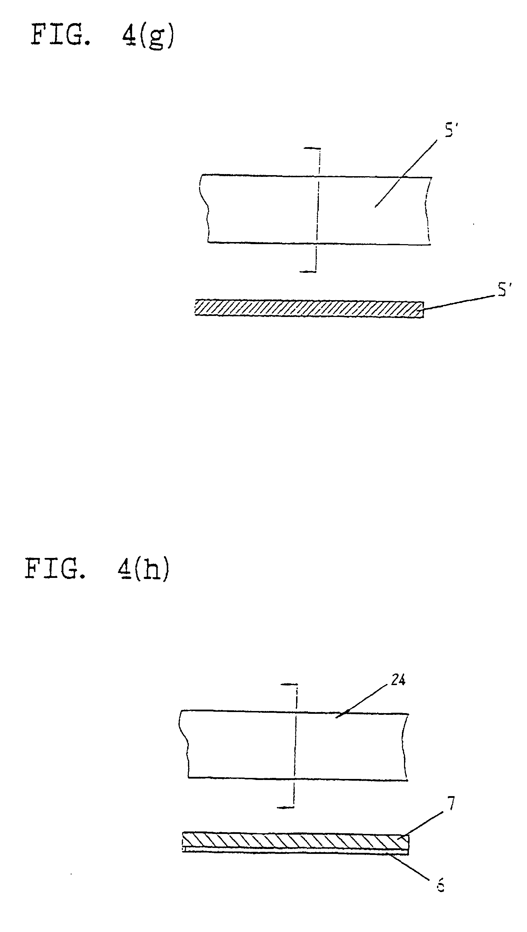

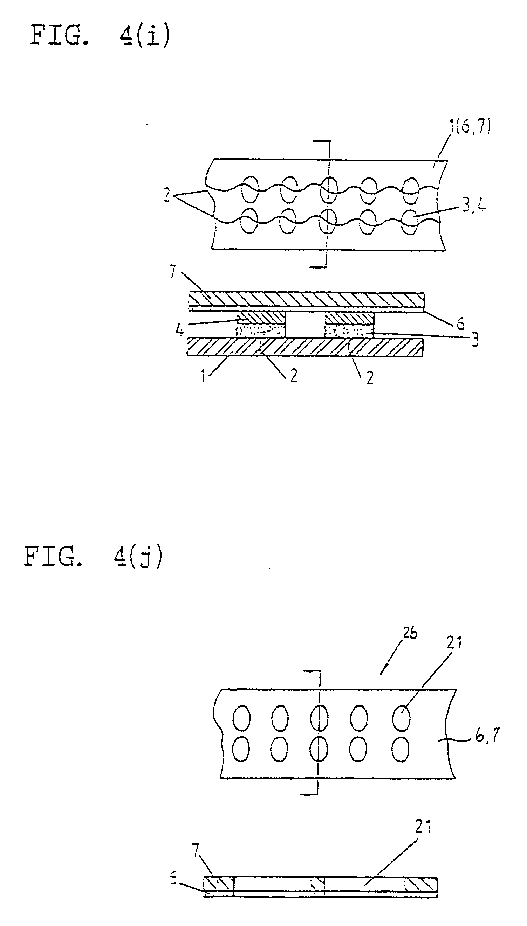

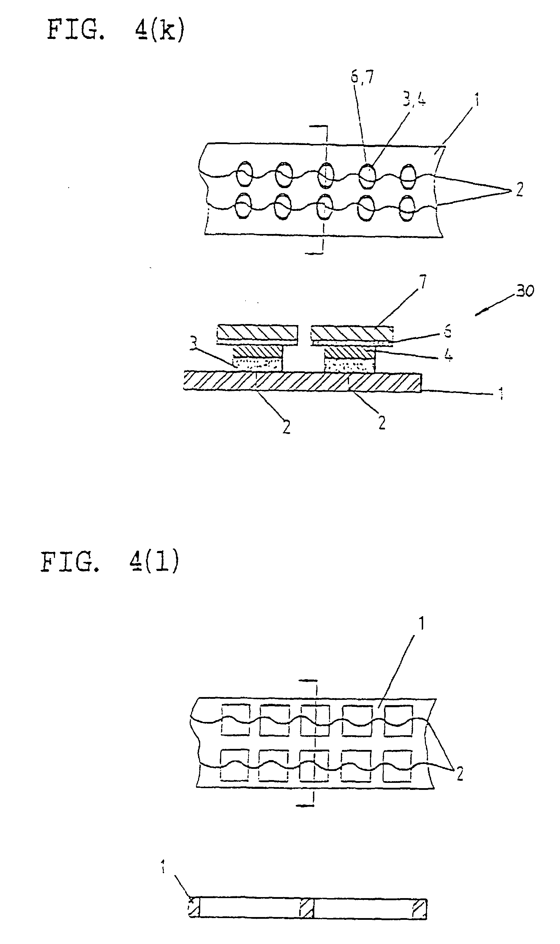

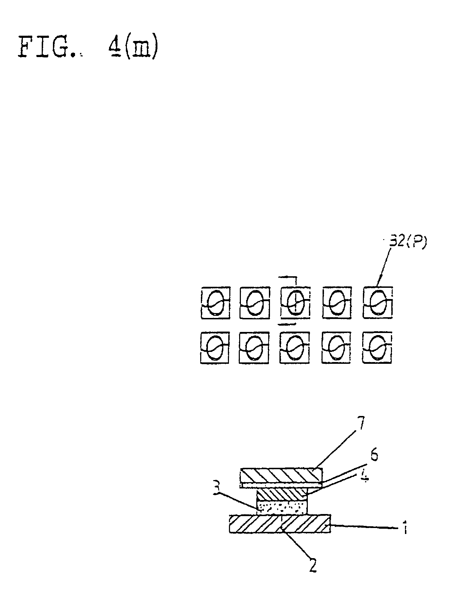

Fig. 3 schematically depicts the steps of manufacturing the medicinal patch for percutaneous administration in accordance with the present invention and Figs. 4(a) to 4(m) are each plan and sectional views of respective base film strip and adhesive tape strip or its/their residual scrap/scraps to be removed in the process of the present invention.

Referring to Figs. 3 to 4(m), the steps of manufacturing the inventive medicinal patch are now fully described. Axis X of Fig. 3 indicates the development of the manufacturing steps in accordance with the present invention.

### FIRST STEP

The base film 10 which is provided with a form of roll and whose structure is a lamination of the separate liner 1, the medicament layer 3, the first elastic film layer 4 and the first release liner 5 in order is drawn out from one of first feed rollers 12 and 12' while the first release liner 5 is peeled off from the base film 10 for removal and wound around its winding roller 60 (Hereinafter the base film with no release liner 5 being preferably called 'released base film'). This step can be referred to Figs. 3, 4(a) and 4(b).

### SECOND STEP

The released base film 10, being unwound from one of first feed rollers 12 and 12', passes through a pair of first half-die cutting rollers 14 and 14' so that at least one wave-shaped cut-and-tear line 2 is consecutively formed only on the separate liner 1 along the length of the released base film 10 as depicted in Figs. 3 and 4(c).

In the medicinal patch for percutaneous administration manufactured by using the inventive apparatus, each of the medicament layer 3 and the elastic film layers 4 and 7 is no more than a few tens of micrometers in thickness and the overall medicinal patch P is so thin that it is not easy to remove the separate liner 1 from the patch unless appropriate cut-and-tear line is provided therein when applying the patch to the diseased region of the body. Therefore, the second step is carried out for consecutively forming at least one cut-and-tear line 2 just on the separate liner 1 by the half-die cutting technique.

### THIRD STEP

The medicament layer 3 and the first elastic film layer 4, overlapped on the separate liner 1 of the base film 10, are simultaneously half-die cut into an oval or round shape on a main die roller 60 through second half-die cutting. A scrap 16 of the medicament layer 3 and the first elastic film layer 4 created from the second half-die cutting is wound on a roller 66 to be removed from the separate liner 1, as shown in Figs. 3 and 4(d) and 4(e).

A first resultant strip 18 where the medicament layer 3 and the first elastic film layer 4 are remained in the shape of oval or circle on the separate liner 1 by said second half-die cutting and its consequent removal of the scrap 16, is transferred forward while maintaining close contact to the outer surface of the main vacuum die roller 60.

### FOURTH STEP

The adhesive tape 20 which is prepared as a roll and whose structure is a lamination of the second release liner 5', the adhesive layer 6 and second elastic film layer 7 is continuously provided from a second feed roller 22 while its second release liner 5' being removed therefrom and collected on its winding roller 70 and, thus, makes a released adhesive tape 24 with no release liner 5'. After being peeled off the second release liner 5', the released adhesive tape 24 is then attached over the first resultant strip 18 where the medicament layer 3 and the first elastic film layer 4 are remained in the shape of oval or circle on the separate liner 1, which is well depicted in Figs. 3 and 4(f), 4(g), 4(h) and 4(i).

The fourth step is also carried out on the main die roller 60 with the help of pressing rollers 56 and 58 which give attaching pressure to the first resultant strip 18 against the main die roller 60 right after the released adhesive tape strip 24 is laid over the first resultant strip 18.

### FIFTH STEP

From this step, the released adhesive tape 24 which has been attached over the first resultant strip 18 are manipulated to have the coincidental oval or circular shape just on the corresponding oval or circular medicament layer 3 and the first elastic film layer 4 by a third half-die cutting. However, it should be noted that the oval or circular regions of the adhesive tape 24 have the same shapes as those of the medicament layer 3 and the first elastic film layer 4 of the first resultant strip 18 but are a little larger than the medicament layer 3 and the first elastic film layer 4 so as to let the differentiated larger area have a role of adhering capability to the skin. A scrap 26 of the released adhesive tape produced at this point is removed by being wound on a roller 74, thus, creating a second resultant strip 30. This process can be well referred to in Figs. 3, 4(j) and 4(k).

The second resultant strip 30 which has the consecutive separate liner 1 with at least one cut-and-tear line 2 therein, the ovally or circularly shaped medicament layer 3 and first elastic film layer 4 lied on said separated liner 1 and the ovally or circularly shaped adhesive layer 6 and second elastic film layer 7 lied just on said ovally or circularly shaped medicament layer 3 and first elastic film layer 4 is drawn up and transferred forward.

### SIXTH STEP

Referring to Fig. 4(l), a die cutting is performed on the second resultant strip 30 into a predetermined shape (e.g. square shape), thus, producing a third resultant strip 32. At this stage, a scrap 86 of the separate liner 1 created from this die cutting is finally removed. The third resultant strips 32 make finished products of medicinal patch prior to packing for sale.

### SEVENTH STEP

The finished products of the third resultant strip 32 are drawn up and transferred from the main die roller 60 to a conveyor 62 by a vacuum pad roller 90.

The method of making the medicinal patch for percutaneous administration comprises the above-described seven steps and the present invention relates to a main unit to perform particularly from the third to seventh steps in the above.

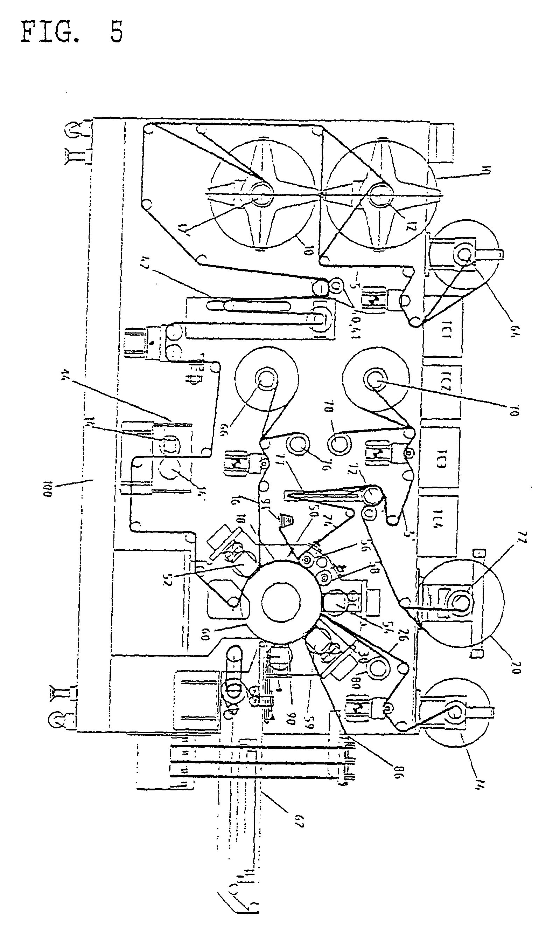

Fig. 5 is a front view of an apparatus for performing said manufacturing steps for manufacturing medicinal patch for percutaneous administration in accordance with the present invention.

Reference numeral 100 denotes a frame of the apparatus for manufacturing the inventive medicinal patch P which is movable with a jack or jacks(not drawn), and made preferably from stainless steel. The first feed rollers 12 and 12' for mounting the roll of base film 10 and drawing it out are rollingly mounted on the frame up and down, so one of them unwinds the base film 10 selectively. If the base film roll mounted on any one of the upper and lower rollers 12 and 12' is all run out, a spare base film roll provided on the other feed roller starts to be drawn out without delay, which saves the loading time to enhance the productivity. These rollers 12 and 12' include a controller to minutely control the base film roll's mounting condition, a hand brake, a device for alarming decrement of the base film, and a sensor for detecting the cutting thereof(not shown).

A pair of rollers 40 and 41 which are so provided as to transfer the base film 10 control their rolling speed according to displacement of a dancer roller 42 having a balancer, and assures the constant-speed transfer of the base film. The dancer roller 42 keeps a predetermined weight by the balancer, and transfers the base film 10 to the main die roller 60 under a given tension regardless of the remained diameter of the base film roll wound around the feed roller 12 or 12'.

A cutting apparatus 44 which comprises a pair of the first die cutting rollers 14, 14' serves to half-die cut only the separate liner 1 of the base film 10 into a waveform. As the base film 10 passes between a pair of the first half-die cutting rollers 14 and 14' (first half-die cutting rollers), the separate liner 1 is half-die cut to have at least one continuous wave-shaped line 2 along its longitudinal direction.

Reference numeral 50 denotes a main unit of the apparatus for making the inventive medicinal patch. By the main unit 50 are performed the following five steps: one is the second half-die cutting by which the medicament layer 3 and the first elastic film layer 4 which are laminated over the separated liner 1 are processed into an oval or round shape; another is attaching the released adhesive tape 24 to the first resultant strip 18; the next is is the third half-die cutting the adhesive tape into an oval or round shape; the next is performing a die cutting the resultant strip 32 to a predetermined shape (e.g. square shape) to produce finished products of medicinal patch P ; and the last is finally drawing up the finished products by the vacuum pad roller 90 and moving them to the conveyor 62.

In reading the present specification, half-die cutting means that, when the sectional structure of a certain strip is made from more than two layers laminated, only the intended or predetermined layer/layers is/are to be cut among the laminated layers, whereas die-cut means to cut all the layers of the section at once. In connection with this, half-die cutting roller means a kind or piece of machine to perform half-die cut and die-cutting roller means a kind or piece of machine to perform die-cut.

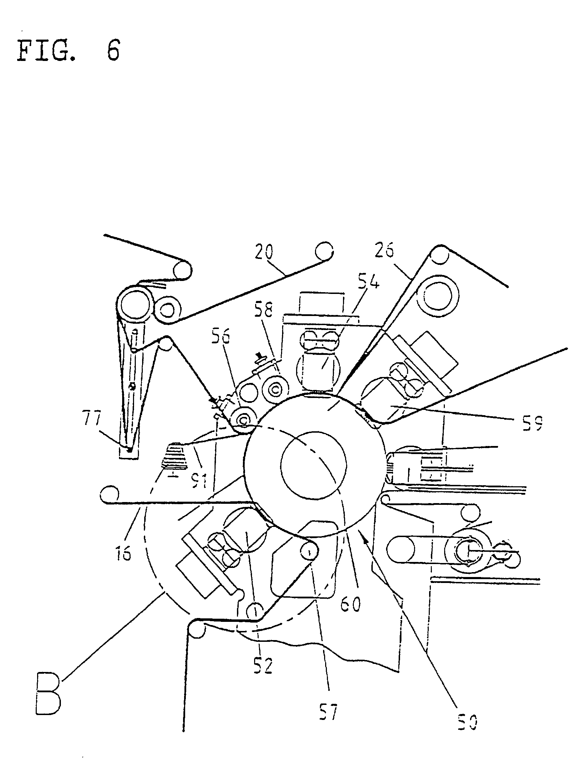

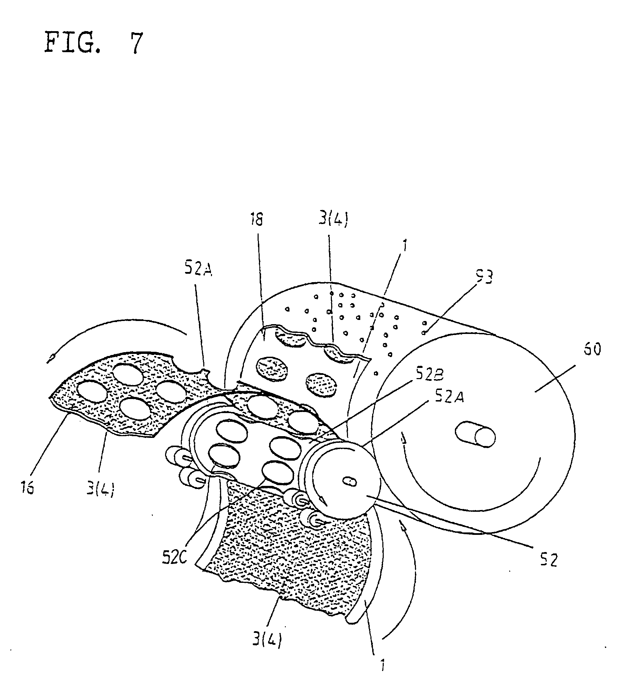

A second half-die cutting roller 52 for half-die cutting only the medicament layer 3 and the first elastic film layer 4 into an oval or round shape, and a third half-die cutting roller 54 for half-die cutting only the adhesive tape into an oval or round shape, each have a plurality of oval or round-shaped kiss cutting blades equidistantly formed along their outer surfaces. A die cutting roller 59 also has a plurality of predetermined shaped, for example, quadri-shaped kiss cutting blades formed equidistant from one another at its outer surface and the detailed structure or configuration of the die cutting roller 59 can be seen in Fig. 7 which shows a perspective view of the part B shown in Fig. 6.

A plurality of vacuum holes(not drawn) are formed on the outer surface of the main die roller 60 for drawing up and transferring the base film 10 during the second, third half-die cutting steps and final die cutting step.

The preliminary and main pressure rollers 56 and 58 used for adhering the adhesive tape 24 over the first resultant strip 18 are interposed between the second half-die cutting roller 52 and the third half-die cutting roller 54. The preliminary pressure roller 56 is an auxiliary one, and the roller 58 is a main one. The process of rolling and compressing the released adhesive tape strip 24 against the first resultant strip 18 on the main die roller 60 prevents bonding failure(for example, air gap entrapped between the base film and the adhesive tape).

The vacuum pad roller 90 has rubber suckers(not drawn) on its outer surface for drawing up the finished products of medicinal patch from the surface of the main die roller 60 by suction, and transfers them to the conveyor 62.

During performing the process described in the above, the first release liner 5 of the base film 10 is collected on a first release liner winding roller 64 under a constant tension with the help of a powder clutch(not drawn) and a tension controller (TC1) regardless its winding diameter.

Reference numeral 66 denotes a roller on which a scrap 16 of the medicament layer 3 and the first elastic film layer 4 is wound. The scrap 16 is wound on the first release liner winding roller 66 under a constant tension with the help of a powder clutch(not drawn) and a tension controller (TC2) regardless of the diameter of the scrap 16 wound.

The adhesive tape roll 20 is the second feed roller 22. Reference numeral 70 is a second release liner winding roller with a single-end fixing type shaft on which the second release liner 5' is collected after being separated from the adhesive tape 20.

An alarming system is provided to the second release liner winding roller 70 for informing an operator of the second release liner 5's being fully wound thereon. The winding tension is constantly kept by a powder clutch(not drawn) and a tension controller (TC3) when winding the release liner 5'.

Reference numeral 74 denotes a roller on which the scrap 26 of the adhesive tape, produced after the third half-die cutting, is wound. An alarming system is also provided to the roller 74, thus informing the operator of the scrap 26's being fully wound on the roller 74. The tension of the strap 26 is constantly maintained by a powder clutch (not drawn)and a tension controller (TC4) when winding the scrap 26 on the roller 74.

A peeling roller 72 has a plurality of vacuum holes(not drawn) on its outer surface for drawing up the adhesive tape strip while separating the second release liner 5' from the adhesive tape strip 20. The adhesive tape strip, from which the second release liner 5' is separated by the peeling roller 72, becomes free from its beared tension by a dancer roller 77 having a given weight kept by a balancer, and avoids its gradual shrinking due to residual tensile stress when the released adhesive tape strip 24 is attached to the first resultant strip 18.

Reference numerals 76, 78, and 80 designate temporarily winding rollers, and these are positioned adjacent to the scrap winding roller 66, the second release liner winding roller 70, and the roller 74 on which the scrap 26 of the adhesive tape is wound, respectively. The rollers 76, 78, and 80 are used only when the scraps are fully wound on the rollers 66, 70, and 74, respectively and so the used ones are to be replaced with the new empty ones. All the rollers 76, 78, and 80 have a same role to save loading or replacing time. These rollers 76, 78, and 80 are preferably configured for non-viscidity to prevent the scraps from sticking to the outer surface of each of them.

Ovally or circularly punched portions 21 are serially formed on the scrap 26 of the released adhesive tape 24 by the third half-die cutting, as shown in Fig. 4(j), and in particular, its tensile strength becomes considerably lessen so that the scrap 26 is easily broken during its winding. Once the scrap 26 is broken, the apparatus cannot perform the next steps, thus lowering the productivity.

In connection with this, reference numeral 91 denotes a means for increasing the tensile strength of the scrap 26 and preventing cutting of the scrap 26 during winding after the adhesive tape 24 is adhered to the first resultant strip 18. Preferably, the tensile stress reinforcing means is realized by thread or string provided to regions of the both longitudinal edges and/or middle portion of the punched scrap 26 of the adhesive tape 24.

Fig. 6 shows an enlarged main part of the apparatus for manufacturing medicinal patch for percutaneous administration in accordance with the present invention, which corresponds to the part A of Fig. 5.

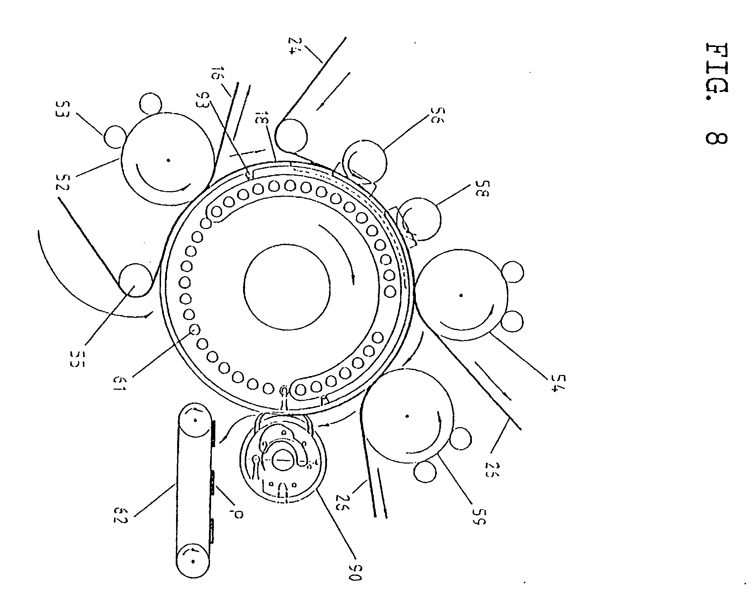

Fig. 7 is a perspective view of the second half-die cutting roller(part B of Fig. 6) in order to show structures of the second and third half-die rollers 52, 54 and die-cutting roller 59(these three being substantially identical). Fig. 8 depicts sectional views of the main die roller 60 and vacuum pad roller 90 to show air passage 61 and vacuum holes 93 for vacuum sucking the patches from the main die roller.

From these Figs. 6 to 8, it can be understood that the second half die cutting roller 52 comprises large-diameter parts 52A formed on both end; a small-diameter part 52B formed between said large-diameter parts; and a plurality of ovally or roundly shaped kiss cutting blades 52C equidistantly installed on outer surface of said small-diameter part, the height of said kiss cutting blades being a little lower than that of the differenc made by said large-diameter parts and small diameter part. Further, it can be also understood that since there are provided a plurality of vacuum holes 93 on the surface of the main die roller, the strip can be transferred in the condition of its close contact to the outer surface of the main die roller during processing steps of said second and third half-die cutting and die-cutting. The strip, under being transferred, is to pass the gaps formed by said difference of heights between the large-diameter parts and small-diameter part of said half-die cutting rollers and die cutting roller and, thus, is to be half-die cut or die cut by said kiss cut blades installed on the small diameter surface.

The following description relates to the operation of the apparatus for making the inventive medicinal patch P.

The base film 10, fed from one of the first feed rollers 12 and 12' at a required constant speed by a pair of transferring rollers 40 and 41, passes between the first half-die cutting rollers 14 and 14' so that only the separate liner 1 of the base film 10 is continuously half-die cut to have at least one wave-shaped cutting line 2(the first half-die cutting).

After the first half-die cutting, the released base film travels the outer surface of the main die roller 60 by closely vacuum contact thereon in accordance with the rolling movement of the main die roller and, then, the medicament layer 3 and the first elastic film layer 4 of the base film 10 are cut out by the oval or round kiss cutting blades equidistantly formed along the outer surface of the second half-die cutting roller 52.

The adhesive tape 24 produced from the adhesive tape roll 20 by peeling off the second release liner 5' therefrom is attached over the first resultant strip 18 with the help of the pressing rollers 56 and 58. In order to give more tensile stress to the scrap 26 of the adhesive tape 24, the thread or string 91 is provided to the back of the adhesive tape 24.

As the second resultant strip 30 formed by attaching the adhesive tape 24 to the first resultant strip 18 is moved a little way along the main die roller 60, the third half-die cutting roller 54, having a plurality of oval- or round-shaped kiss cutting blades equidistantly installed on its outer surface, half-die cuts only the adhesive tape 24 of the second resultant strip into an oval or round shape. A scrap 26 produced from the above process is wound on the roller 74 for removal. At this stage, the third half-die cutting is carried out on the regions of the adhesive tape 24 corresponding to the ovally or circularly shaped medicament and first elastic film layers 3 and 4.

The strip on which the third half-die cutting is performed is moved a little way along the main die roller 60 closely contacting the main die roller 60. The overall strip is then die cut out by the die cutting roller 59 which has a plurality of square-shaped kiss cutting blades formed equidistant from one another at its outer surface, thus producing finished products of medicinal patch P as shown in Fig. 1. The vacuum pad roller 90 draws up each finished product with its rubber suckers that are circumferentially formed equidistantly from each other, and transfers it to the conveyor 62. Automatic inspection and packing are performed on each finished product P of medicinal patch on the conveyor 62.

As described above, medicinal patch for percutaneous administration made by the apparatus according to the manufacturing steps of the present invention can fulfill the physical requirements of a transdermal skin patch. That is, the inventive medicinal patch can adhere to the skin of the knee or elbow securely and elastically for a long period of time even during bath or shower bath, and is not easily contaminated by the dirt without loss of medicinal properties. In addition, the oval- or round-shaped medicinal patch of the present invention is so thin and small that it can be securely attached to the skin of any diseased region of the human body, thus being superior to the presently-available transdermal skin patch.

Additional advantages and modifications will readily occur to those skilled in the art. The invention in its broader aspects is not limited to the specific details, and representative devices, shown and described herein. Accordingly, various modifications may be made without departing from the scope of the general inventive concept as defined by the appended claims and their equivalents.

## Claims

1. An apparatus for manufacturing a medicinal patch for percutaneous administration comprising:
a half-die cutting roller(a second half-die cutting roller) (52) to ovally or roundly half die cut the medicament layer (3) and first elastic film layer (4) simultaneously laminated on a separate liner of a base film, said half-die cutting roller having large-diameter cylindrical parts, a small-diameter cylindrical part formed between said large-diameter cylindrical parts and a plurality of ovally or roundly shaped kiss cutting blades equidistantly installed on the outer surface of said small-diameter cylindrical part;
a scrap winding roller around which a scrap of the medicament and first elastic film layers produced by half-die cutting is wound for removal;
a pressing means to press the adhesive tape laminated the base film on which ovally or roundly shaped medicament and first elastic film layers are formed by the second half-die cut so as to attach these with each other;
another half-die cutting roller(a third half-die cutting roller) (54) which has a plurality of ovally or roundly half-die cutting blades equidistantly installed on its outer surface of small-diameter cylindrical part, performing continuous half-die cutting on regions of the adhesive tape attached to the base film into an oval or round shape, corresponding to the second half-die cut regions of the medicament and first elastic film layers;
a roller on which a scrap of the adhesive tape produced by the third half-die cutting is wound for removal;
a die cutting roller which has a plurality of quadri-shaped die cutting blades equidistantly installed on its outer surface, performing a continuous die cutting of a resultant strip which has the separate liner, the oval or circular shaped medicament and first elastic film layers and the oval or circular shaped adhesive and second elastic film layers, into a quadri-shaped shape;
a roller on which a scrap of the resultant strip created by the die cutting is wound for removal;
a main die roller (60) having a plurality of vacuum holes on its outer surface for drawing up the separate liner of the base film (10), and with the outer surface thereof large diameter parts of all the half die cut rollers and die cut roller being in rollably contact; and,
a vacuum pad roller (90) having a plurality of rubber sucking means on its outer surface for drawing up the finished products from the main die roller (60) and transferring them to a conveyor (62).

2. An apparatus according to claim 1, wherein said pressing means comprises a pair of roller interposed between the second and third half-die cutting rollers, one of them being a preliminary pressure roller (56) and the other being a main pressure roller (58).

3. An apparatus according to claim 1 or 2, further comprising means for adding more tensile strength to the scrap (26) of the adhesive tape when it is removed after the third half-die cutting.

4. An apparatus according to claim 3, wherein said tensile strength reinforcing means is a thread or string lengthwise provided to both longitudinal edges and/or middle portion of the adhesive tape.

5. An apparatus according to claim 1, further comprising temporarily winding rollers, each of which being adjacent to said medicament scrap winding roller, said second release liner winding roller (70), and said roller (74) to wind the scrap (26) of the adhesive tape respectively and used temporarily only when the scraps are fully wound on said three rollers.

## Patentansprüche

1. Vorrichtung zur Herstellung eines medizinischen Pflasters zur perkutanen Verabreichung, umfassend
- eine Halb-Schneideisen-Schneidwalze (eine zweite Halb-Schneideisen-Schneidwalze (52)), um in ovaler oder runder Form mit dem Schneideisen halb die ein Medikament umfassende Schicht (3) und die Schicht (4) aus einem ersten elastischen Film auszuschneiden, die gleichzeitig auf einer getrennten Liner-Schicht eines Grund-Films laminiert sind, wobei die Halb-Schneideisen-Schneidwalze zylindrische Teile mit einem großen Durchmesser, einen zylindrischen Teil mit einem kleinen Durchmesser, der zwischen den zylindrischen Teilen mit einem großen Durchmesser gebildet ist, und eine Mehrzahl oval oder rund geformter Kiss-Schneidmesser aufweist, die in gleichem Abstand zueinander auf der Außenoberfläche des zylindrischen Teils mit dem kleinen Durchmesser angeordnet sind;
- eine Verschnitt-Aufwickelwalze, um die ein Verschnitt der ein Medikament umfassenden Schicht und der Schicht aus einem ersten elastischen Film, der durch das Schneiden mit dem Halb-Schneideisen erzeugt wird, zum Entfernen aufgewickelt wird;
- eine Preß-Einrichtung, um das Klebeband laminatartig auf den Grund-Film zu pressen, auf dem oval oder rund geformte Medikament-Schichten und Schichten aus einem ersten elastischen Film durch den Schnitt mit dem zweiten Halb-Schneideisen gebildet sind, um diese aneinander zum Haften zu bringen;
- eine weitere Halb-Schneideisen-Schneidwalze (eine dritte Halb-Schneideisen-Schneidwalze (54)), die eine Mehrzahl oval oder rund geformter Halb-Schneideisen-Schneidmesser aufweist, die in gleichem Abstand zueinander auf der Außenoberfläche ihres zylindrischen Teils mit dem kleinen Durchmesser angeordnet sind und ein kontinuierliches Halb-Schneideisen-Schneiden zu einer ovalen oder runden Form, die den Bereichen des zweiten Halb-Schneideisen-Schnitts der Medikamenten-Schicht und der Schicht des ersten elastischen Films entspricht, in Bereichen des Klebebandes durchführen, die an dem Grund-Film haften;
- eine Walze, auf der ein Verschnitt des Klebebandes zum Entfernen aufgewickelt wird, der durch das Schneiden mit dem dritten Halb-Schneideisen erzeugt wird;
- eine Schneideisen-Schneidwalze, die eine Mehrzahl von quadratisch geformten Schneideisen-Schneidklingen aufweist, die in gleichem Abstand auf ihrer Außenoberfläche angeordnet sind und die ein kontinuierliches Schneideisen-Schneiden eines resultierenden Streifens, der den getrennten Liner aufweist, der ovalen oder kreisförmig geformten Medikamenten-Schicht und Schicht aus einem ersten elastischen Film und der ovalen oder kreisförmig geformten Haftschicht und Schicht aus einem zweiten elastischen Film in eine quadratisch geformte Form durchführt;
- eine Walze, auf die ein Verschnitt des resultierenden Streifens zum Entfernen aufgewickelt wird, der durch das Schneideisen-Schneiden erzeugt wird;
- eine Haupt-Schneidwalze (60), die eine Mehrzahl von Vakuum-Löchern auf ihrer Außenoberfläche zum Abziehen des separaten Liners von dem Grund-Film (10) aufweist und mit deren Oberfläche die Teile aller Halb-Schneideisen-Schneidwalzen mit großem Durchmesser und die Schneideisen-Schneidwalze in Roll-Kontakt stehen; und
- eine Vakuum-Pflaster-Walze (90), die eine Mehrzahl von Saug-Einrichtungen aus Kautschuk auf ihrer Außenoberfläche zum Abziehen der fertigen Produkte von der Haupt-Schneidwalze (60) und deren Überführung auf eine Fördereinrichtung (62) aufweist.

2. Vorrichtung nach Anspruch 1, worin die Preß-Einrichtung ein Walzenpaar umfaßt, das zwischen der zweiten und der dritten Halb-Schneideisen-Schneidwalze angeordnet ist, wobei eine der Walzen des Walzenpaars eine Vor-Druck-Walze (56) und die andere eine Haupt-Druck-Walze (58) ist.

3. Vorrichtung nach Anspruch 1 oder Anspruch 2, umfassend weiter eine Einrichtung zum Aufbringen von mehr Zugfestigkeit auf den Verschnitt (26) des Klebebandes, wenn dieses nach dem Schneiden mit dem dritten Halb-Schneideisen entfernt wird.

4. Vorrichtung nach Anspruch 3, worin die Einrichtung zur Verstärkung der Zugfestigkeit ein Faden oder eine Schnur ist, die an beiden Längskanten und/oder im Mittelabschnitt des Klebebandes in Längsrichtung vorgesehen ist.

5. Vorrichtung nach Anspruch 1, umfassend weiter vorübergehend aufwickelnde Walzen, von denen jede benachbart ist der Aufwickel-Walze des Verschnitts der Medikamenten-Schicht, der Aufwickel-Walze (70) der zweiten Schutzpapier-Schicht bzw. der Walze (74) zum Aufwickeln des Verschnitts (26) des Klebebandes und die nur dann vorübergehend verwendet werden, wenn die Verschnitte voll auf den drei Walzen aufgewickelt sind.

## Revendications

1. Un dispositif de fabrication d'un pansement médicinal pour administration percutanée, comprenant :
un rouleau de découpage à demi-matrice (un deuxième rouleau de découpage à demi-matrice) (52) pour découper par matriçage une moitié, ovale ou ronde, de la couche médicamenteuse (3), et une première couche de film (4) élastique, simultanément laminée sur un revêtement séparé d'un film de base, ledit rouleau de découpage à demi-matrice ayant des parties cylindriques de grand diamètre, une partie cylindrique de petit diamètre formée entre lesdites parties cylindriques de grand diamètre, et une pluralité de lames de découpage par effleurage, de forme ovale ou ronde, installée de façon équidistante sur la surface extérieure de ladite partie cylindrique de petit diamètre ;
un rouleau d'enroulement de morceaux autour duquel un morceau des couches médicamenteuses et de première couche de film élastique, produit par le découpage à demi-matrice, est enroulé pour enlèvement ;
des moyens de pressage, pour presser le ruban adhésif, laminé avec le film de base sur lequel sont formées des couches médicamenteuses à forme ovale ou ronde, et des premières couches en film élastique, par le deuxième découpage à demi-matrice, pour fixer ces éléments les uns aux autres ;
un autre rouleau de découpage à demi-matrice (un troisième rouleau de découpage à demi-matrice) (54), comportant une pluralité de lames de découpage à demi-matrice, ovales ou rondes, installées de façon équidistante sur sa surface extérieure de la partie cylindrique à petit diamètre, permettant d'effectuer un découpage à demi-matrice continu sur des régions du ruban adhésif fixé sur le film de base, à une forme ovale ou ronde, correspondant aux deuxièmes régions de découpage à demi-matrice des couches médicamenteuses et premières couches de film élastique ;
un rouleau sur lequel un morceau du ruban adhésif produit par le troisième découpage à demi-matrice est enroulé pour enlèvement ;
un rouleau de découpage à matrice, ayant une pluralité de lames de découpage à matrice à forme quadrilatérale, installées de façon équidistante sur sa surface extérieure, effectuant un découpage à la matrice continu d'une bande résultante comportant le revêtement séparé, les couches médicamenteuses à forme ovale ou circulaire et les premières couches de film élastique, et les couches d'adhésif à forme circulaire et les deuxièmes couches de film élastique, à une forme de quadrilatère ;
un rouleau, sur lequel un morceau de la bande résultante créée par le découpage à la matrice est enroulée pour enlèvement ;
un rouleau de matrice principal (60) ayant sur sa surface extérieure une pluralité de trous à vide pour aspirer le revêtement séparé d'un film de base (10), et des parties de grand diamètre de la totalité des rouleaux de découpage à demi-matrice et le rouleau de découpage à matrice étant en contact de roulement avec la surface extérieure de celui-ci ; et
un rouleau de patin à vide (90) ayant sur sa surface extérieure une pluralité de moyens d'aspiration ou ventouses en caoutchouc pour aspirer les produits finis depuis le rouleau de matrice principal (60) et les transférer à un convoyeur (62).

2. Un dispositif selon la revendication 1, dans lequel lesdits moyens de pressage comprennent une paire de rouleaux interposés entre les deuxièmes et troisièmes rouleaux de découpage à demi-matrice, l'un d'entre eux étant un rouleau à pression préliminaire (56) et l'autre étant un rouleau à pression principale (58).

3. Un dispositif selon la revendication 1 ou 2, comprenant en outre des moyens pour ajouter plus de résistance la traction au morceau (26) du ruban adhésif lorsqu'il est enlevé après le troisième découpage à demi-matrice.

4. Un dispositif selon la revendication 3, dans lequel lesdits moyens de renforcement de résistance à la traction sont formés d'un filet ou d'une chaîne, prévu dans la direction longitudinale sur les deux bords longitudinaux et/ou sur la partie médiane du ruban adhésif.

5. Un dispositif selon la revendication 1, comprenant en outre des rouleaux d'enroulement temporaire, dont chacun est adjacent audit rouleau d'enroulement de morceaux médicamenteux, audit deuxième rouleau d'enroulement de revêtement de détachement du pansement (70) et audit rouleau (74), pour enrouler le morceau (26) de ruban adhésif, respectivement, et utilisés temporairement uniquement lorsque les morceaux sont complètement enroulés sur lesdits trois rouleaux.
